(19) Europäisches Patentamt
European Patent Office
Office européen des brevets

(11) Veröffentlichungsnummer: **0 002 680**
**B1**

(12) **EUROPÄISCHE PATENTSCHRIFT**

(21) Anmeldenummer: **78101542.5**

(22) Anmeldetag: **04.12.78**

(51) Int. Cl.³: **C 07 D 493/04**, A 61 K 31/335//(C07D493/04, 319/00, 311/00)

(54) **4-Spectinomycylamin und seine physiologisch verträgliche Salze, Verfahren zur Herstellung und diese Stoffe enthaltende Arzneimittel.**

(30) Priorität: **21.12.77 DE 2756914**

(43) Veröffentlichungstag der Anmeldung:
**11.07.79 Patentblatt 79/14**

(45) Bekanntmachung des Hinweises auf die Patenterteilung:
**11.02.81 Patentblatt 81/6**

(84) Benannte Vertragsstaaten:
**BE CH DE FR GB IT LU NL SE**

(56) Entgegenhaltungen:
**US - A - 3 819 485**

(73) Patentinhaber: **Dr. Karl Thomae GmbH**
**Postfach 720**
**D-7950 Biberach (Riss) (DE)**

(72) Erfinder: **Maier, Roland, Dipl.-Chem. Dr.**
**Eichendorffweg 36**
**D-7950 Biberach 1 (DE)**

(72) Erfinder: **Woitun, Eberhard, Dipl.-Chem. Dr.**
**Stecherweg 17**
**D-7950 Biberach 1 (DE)**

(72) Erfinder: **Reuter, Wolfgang, Dipl.-Chem. Dr.**
**D-7951 Laupertshausen 139 (DE)**

(72) Erfinder: **Wetzel, Bernd, Dipl.-Chem. Dr.**
**Ginsterhalde 9**
**D-7950 Biberach 1 (DE)**

(72) Erfinder: **Goeth, Hanns, Dr.**
**Oberer Bühl 6**
**D-7950 Biberach 1 (DE)**

(72) Erfinder: **Lechner, Uwe, Dr.**
**Panoramastrasse 12**
**D-7951 Ummendorf (DE)**

**EP 0 002 680 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 4-Spectinomycylamin und seine physiologisch verträgliche Salze, Verfahren zur Herstellung und diese Stoffe enthaltende Arzneimittel

Die Erfindung betrifft 4-Spectinomycylamin der Formel

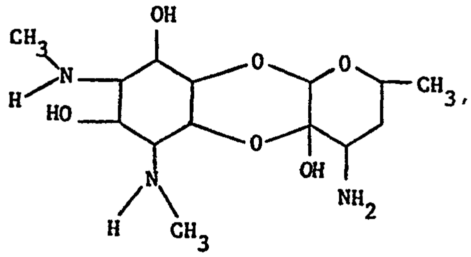

(I)

dessen physiologisch verträgliche Säureadditionssalze mit anorganischen oder organischen Säuren, Verfahren zu deren Herstellung und diese Stoffe enthaltende Arzneimittel.

Das 4-Spectinomycylamin der Formel I kann auch als Decahydro-4a,7,9-trihydroxy-4-amino-2-methyl-6,8-bis(methylamino)-pyrano[2,3-b][1,4]benzodioxin beschrieben werden. Diese Verbindung besitzt eine ausgezeichnete antimikrobielle Wirksamkeit bei guter Verträglichkeit und ist damit dem bekannten Spectinomycin (früher als Actinospectacin bezeichnet) als Breitband-Antibiotikum überlegen.

Die Herstellung des 4-Spectinomycylamins erfolgt wie nachstehend beschrieben:

1. Durch Reduktion eines Spectinomycinderivates der allgemeinen Formel

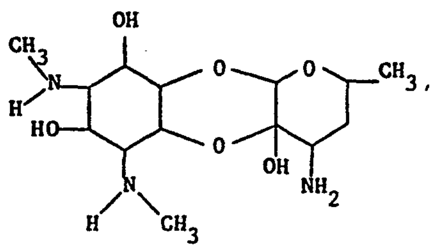

(II)

in der X und Y folgende Bedeutungen haben:

X ein Wasserstoffatom, eine aus der Peptidchemie bekannte, durch Reduktion leicht abspaltbare organische Gruppe, wie die Benzyloxycarbonylgruppe, die 4-Brom- oder 4-Nitro- oder 4-Chlor-benzyloxycarbonylgruppe, die 4-Methoxy- oder 3,4-Dimethoxy- oder 3,4-Methylen-dihydroxy- oder 3,4,5-Trimethoxy- oder 4-Decyloxy- oder 4-Acetoxy- oder 4-Äthoxycarbonyloxy-benzyloxycarbonylgruppe, eine Phenyl- oder Biphenylalkoxycarbonylgruppe, die im Phenylrest durch eine bis drei Methyl- oder Methoxygruppen substituiert sein kann und deren Alkylengruppe, die gerade oder verzweigt sein kann, 2 bis 4 Kohlenstoffatome enthält, wie zum Beispiel die $\alpha,\alpha$-Dimethyl-3,5-dimethoxy-benzyloxycarbonyl- oder 2-[Biphenylyl-(4)]-propyl-(2)-oxycarbonylgruppe, die gegebenenfalls durch eine Nitro-, Methoxy oder Methylgruppe substituiert sein kann, eine Dialkylaminooxycarbonylgruppe, wie die Dimethylaminooxycarbonylgruppe oder die Piperidinooxycarbonylgruppe, oder auch eine Benzyl- oder Tritylgruppe und

Y eine Hydroxygruppe, eine Alkoxygruppe mit 1 bis 10 Kohlenstoffatomen, die Phenalkoxygruppe mit insgesamt 7 bis 12 Kohlenstoffatomen oder eine Gruppe der Formel

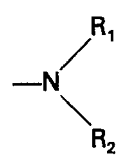

in der $R_1$ und $R_2$ Wasserstoffatome, Alkylgruppen mit 1 bis 6 Kohlenstoffatomen, Phenalkylgruppen mit insgesamt 7 bis 10 Kohlenstoffatomen oder die Phenylgruppe darstellen, wobei $R_1$ auch eine aliphatische Acylgruppe mit 1 bis 10 Kohlenstoffatomen oder die Benzoylgruppe sein kann, sofern dann $R_2$ die anderen vorstehend genannten Bedeutungen innehat, oder eine Gruppe der Formel

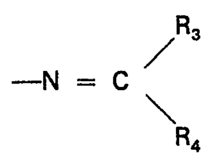

in der $R_3$ und $R_4$ Wasserstoffatome, Alkylgruppen mit 1 bis 6 Kohlenstoffatomen, Phenalkylgruppen mit 7 bis 10 Kohlenstoffatomen oder die Phenylgruppe darstellen, wobei $R_3$ und $R_4$ zusammen mit dem dazwischenliegenden Kohlenstoffatom auch einen 5- bis 8-gliedrigen carbocyclischen Ring bilden kann.

Zur Reduktion eignet sich besonders die katalytische Hydrierung in Gegenwart von Metallkatalysatoren, wie beispielsweise feinverteiltes Platin, Palladium oder Platindioxid. Die Hydrierung wird in Wasser, in organischen Lösungsmitteln, wie Alkoholen, Carbonsäuren, Dioxan, Tetrahydrofuran, oder in Gemischen dieser Lösungsmittel bei Temperaturen zwischen 0° und 100°C, vorzugsweise 20 und 50°C, und bei Drucken zwischen 1 und 100 at durchgeführt.

Vorteilhaft erweist sich dabei die Zugabe von anorganischen oder organischen Säuren, wie Chlorwasserstoff, Bromwasserstoff, Trifluoressigsäure, Methansulfonsäure oder Trifluormethansulfonsäure.

2. Durch Abspaltung der Reste X' aus 4-Spectinomycylaminderivaten der allgemeinen Formel

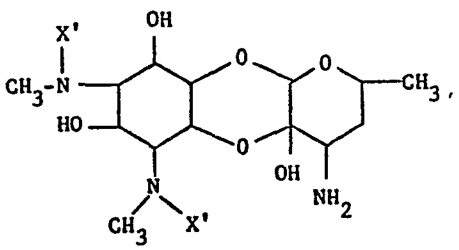 (III)

Darin bedeutet

X' eine aus der Peptidchemie bekannte, durch Behandeln mit Säuren, Basen oder durch Reduktion leicht abspaltbare organische Gruppe, wie die Benzyloxycarbonylgruppe, die 4-Brom- oder 4-Nitro- oder 4-Chlor-benzyloxycarbonylgruppe, die 4-Methoxy- oder 3,4-Dimethoxy- oder 3,4-Methylendihydroxy- oder 3,4,5-Trimethoxy- oder 4-Decyloxy-oder 4-Acetoxy- oder 4-Äthoxy-carbonyloxybenzyloxycarbonylgruppe, eine gesättigte oder ungesättigte Alkoxycarbonylgruppe mit 1 bis 12 Kohlenstoffatomen, die gegebenenfalls durch eine Furyl-(2)-gruppe, eine p-Tolylsulfonylgruppe, ein oder mehrere Halogenatome, eine Alkoxy- oder Alkoxy-alkoxygruppe mit 1 bis 3 Kohlenstaffatomen im Alkylteil und 1 bis 3 Kohlenstoffatomen im Alkylenteil substituiert sein kann, beispielsweise die Furyl-(2)-methoxycarbonyl-, Allyloxycarbonyl-, 2-(p-Tolylsulfonyl)-äthoxycarbonyl-, 2-Brom-äthoxycarbonyl-, 2,2,2-Trichlor-äthoxycarbonyl-, 2-(2-Methoxy-äthoxy)äthoxycarbonyl-, 3-Methyl-pentyl-(3)-oxycarbonyl- insbesondere aber die tert.-Butyloxycarbonylgruppe, eine Cycloalkyloxycarbonylgruppe mit 5 bis 12 Kohlenstoffatomen, wie die Cyclopentyloxycarbonyl- oder Cyclohexyloxycarbonylgruppe, die beide durch eine Methyl-, Äthyl- oder tert.-Butylgruppe substituiert sein können, die Isobornyloxycarbonyl- oder die Adamantyl-(1)-oxycarbonylgruppe, eine Phenyl- oder Biphenylalkoxycarbonylgruppe, die im Phenylrest durch eine bis drei Methyl- oder Methoxygruppen substituiert sein kann und deren Alkylengruppe, die gerade oder verzweigt sein kann, 2 bis 4 Kohlenstoffatome enthält, wie zum Beispiel die $\alpha,\alpha$-Dimethyl-3,5-dimethoxy-benzyloxycarbonyl- oder 2-[Biphenylyl-(4)]-propyl-)2)-oxycarbonylgruppe, die Diphenyl-methoxycarbonylgruppe, eine Phenyloxycarbonylgruppe, die gegebenenfalls durch eine Nitro-, Methoxy oder Methylgruppe substituiert sein kann, eine Dialkylaminooxycarbonylgruppe, wie die Dimethylaminooxycarbonylgruppe oder die Piperidinooxycarbonylgruppe, eine Alkylthiocarbonylgruppe mit 1 bis 4 Kohlenstoffatomen im Alkylrest, die Benzylthiocarbonylgruppe, die Formylgruppe oder eine sonstige aliphatische Acylgruppe mit 1 bis 10 Kohlenstoffatomen, welche gegebenenfalls noch durch 1 bis 3 Halogenatome, Hydroxygruppen, Acylreste, oder durch eine Nitrogruppe substituiert sein kann, wie die Trifluoracetyl-, Acetoacetyl-, 2-Nitro-phenoxy-acetyl-, Monochloracetyl-, 3-Chlor-butyroyl-, 3-Hydroxyisocaproylgruppe, desweiteren kann X eine Benzoyl-, 2-Nitrobenzoyl-, 4-Toluolsulfonyl-, Benzylsulfonyl- oder p-Methoxybenzolsulfonylgruppe oder auch eine Benzyl- oder Tritylgruppe sein.

Die Abspaltung des Restes X' erfolgt beispielsweise mittels katalytischer Hydrogenolyse in wäßrig-organischen oder rein organischen Lösungsmitteln wie Alkohole, Essigsäure, Dimethylformamid in Gegenwart von Edelmetallkatalysatoren wie Palladiumschwarz, Palladiumkohle oder Palladium auf Bariumsulfat, Platin auf Kohle oder durch reduktive Spaltung mit Natrium in flüssigem Ammoniak oder durch eine acidolytische Spaltung mit einer Halogenwasserstoffsäure, wie Chlorwasserstoff, Bromwasserstoff, Trifluoressigsäure oder einer organischen Sulfonsäure in Lösungsmitteln wie Eisessig, Chloroform, Wasser oder Äthanol. Alkoxycarbonylgruppen lassen sich auch durch die Einwirkung von Natriumhydroxid in Wasser-Äthanol-Gemischen oder durch die Einwirkung von Natriumäthanolat in Äthanol entfernen. Die gegebenenfalls substituierten Phenyloxycarbonylgruppen oder Benzyloxycarbonyl- sowie die Dialkylaminooxycarbonylgruppen lassen sich durch katalytische Hydrierung entfernen.

Acylreste lassen sich besonders gut durch die Einwirkung einer methanolischen Salzsäure oder wässerigen Laugen abspalten. Die 2,2,2-Trichlor-äthoxycarbonylgruppe wird vorteilhafterweise durch die Einwirkung von Zinkpulver in Essigsäure oder von Zinkstaub in Methanol entfernt. Die Benzylgruppe wird dagegen bevorzugt durch katalytische Hydrierung mit Palladium, die Tritylgruppe durch saure Verseifung mittels Essigsäure entfernt. Die meisten Gruppen X' lassen sich aber beispielsweise mit Bromwasserstoff in Eisessig bei Temperaturen zwischen 20 und 50°C abspalten.

Das 4-Spectinomycylamin der Formel I kann, gewünschtenfalls, anschließend mittels anorganischer oder organischer Säuren nach bekannten Methoden in seine physiologisch verträglichen Säureadditionssalze übergeführt werden. Als Säuren kommen beispielsweise in Frage: Salzsäure, Bromwasserstoffsäure, Schwefelsäure, Phosphorsäure, Maleinsäure, Zitronensäure, Weinsäure oder Fumarsäure.

Ein als Ausgangssubstanz dienendes Spectinomycinderivat der allgemeinen Formel II läßt sich aus einem Spectinomycinderivat der allgemeinen Formel

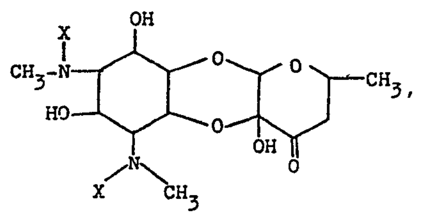

(IV)

in der X wie oben definiert ist, durch Umsetzung mit einer Verbindung der allgemeinen Formel

$NH_2$—Y, (V)

in der Y ebenfalls wie oben definiert ist, herstellen. Die Umsetzung wird in Wasser oder in einem organischen Lösungsmittel, beispielsweise in Alkoholen, wie Äthanol, Isopropanol, in Carbonsäuren, wie Eisessig, in Estern oder Äthern, wie Dioxan oder in Gemischen solcher Lösungsmittel bei Temperaturen zwischen 0° und 100°C, vorzugsweise zwischen 0° und 50°C durchgeführt.

Die als Ausgangssubstanzen dienenden 4-Spectinomycylamin-derivate der allgemeinen Formel III erhält man ebenfalls aus den Verbindungen der allgemeinen Formel IV and zwar dadurch, daß man diese mit Ammoniumsalzen in Gegenwart von Alkalicyanborhydriden umsetzt. Die Umsetzung wird in Wasser oder in organischen Lösungsmitteln, vorzugsweise in Alkoholen, oder in Gemischen dieser, bei Temperaturen zwischen 0° und 100°C, vorzugsweise 0° und 50°C, vorgenommen. Als Ammoniumsalze kommen beispielsweise die Salze des Ammoniaks mit Halogenwasserstoffsäuren, Schwefelsäure, Phosphorsäure und Salpetersäure in Betracht, als Alkalicyanborhydride vorzugsweise das Natriumcyanborhydrid.

Die Ausgangsverbindungen der allgemeinen Formel III, in der X' die oben angegebenen Bedeutungen mit Ausnahme der Bedeutung einer durch Reduktion leicht abspaltbaren Gruppe, besitzt (bei den durch Reduktion abspaltbaren Gruppen handelt es sich um die oben für X der allgemeinen Formel II angegebenen), lassen sich dadurch erhalten, daß Verbindungen der allgemeinen Formel II, in der anstelle von X der Rest X' sitzt, reduziert werden, beispielsweise durch katalytische Hydrierung.

Bei der Herstellung des 4-Spectinomycylamins der Formel I treten zwei isomere Verbindungen auf, nämlich die 4 R-Form mit axialer Aminogruppe und die 4 S-Form mit äquatorialer Aminogruppe. Die 4 R-Form wird als 4-Spectinomycylamin I bezeichnet und zeigt einen Rf-Wert von 0,5, die 4 S-Form, das 4-Spectinomycylamin II, besitzt dagegen einen Rf-Wert von 0,4 (Adsorbens: Kieselgel G, Fließmittel Chloroform: Methanol: konzentriertem Ammoniak wie 40:40:20:). Es ist dadurch leicht möglich, mittels chromatographischer Methoden beide Isomere voneinander zu trennen. Eine Zuordnung der Rf-Werte zu den jeweiligen Isomeren was deshalb möglich, weil diese Isomere verschiedenes chemisches Verhalten beispielsweise bei der Umsetzung mit Aceton oder dem 2-Cyano-3,3-bis(methyl)-mercaptoacrylsäuremethylester zeigten.

Beide Spectinomycylamine besitzen wertvolle antimikrobielle Eigenschaften; besonders ausgeprägt sind die Eigenschaften bei der 4-R-Form, also dem 4-Spectinomycylamin I.

Es wurde das 4R-Spectinomycylamin der Formel I vergleichend mit dem bekannten Spectinomycin bezüglich der Wirkung gegen Staphylococcus aureus SG 511, Streptococcus Aronson, Escherichia Coli ATCC 9637, Pseudomonas aeruginosa, Serratia marcescens ATCC 13 880, Klebsiella pneumoniae ATCC 10 031, Proteus mirabilis und Proteus vulgaris und bezüglich der akuten Toxizität untersucht.

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht: 80; 40;, 20;, 10; 5; 2,5; 1,25; 0,6 und 0,3 µg/ml. Als Nährmedium diente eine Bouillon aus 5 g Pepton, 3 g Fleischextrakt, aufgefüllt mit destilliertem Wasser auf ein Volumen von 1 000 ml; pH-Wert 6,7. Das Alter der Primärkulturen betrug 24 Stunden. Die Einstellung der Keimsuspension erfolgte am Photometer (nach "Eppendorf") (Ragenzglas-Durchmesser 14 mm, Filter 546 nm) an Hand der Trübung einer Bariumsulfat-Vergleichssuspension, die durch eine Bariumsulfat-Aufschwemmung erzeugt wurde, welche durch die Zugabe von 3,0 ml 1%ige Bariumchloridlösung in 97 ml 1%ige Schwefelsäure entstand. Nach der Einstellung

wurden Streptococcus Aronson im Verhältnis 1:150 und die übrigen Testkeime im Verhältnis 1:1 500 mit einer Kochsalzlösung weiter verdünnt.

16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

In der folgenden Tabelle sind die ermittelten Konzentrationen bzw. minimale Hemmkonzentrationen für die beiden obengenannten Substanzen angegeben:

| Keime | Minimale Hemmkonzentration in $\mu$g/ml der Substanz | |
|---|---|---|
| | 4R-Spectinomycylamin | Spectinomycin |
| Staphylococcus aureus SG 511 | 5 (2,5) | 20 (10) |
| Streptococcus Aronson | 5 | 10 |
| Escherichia Coli ATCC 9637 | 20 (10) | 40 (20) |
| Pseudomonas aeruginosa | 40 (20) | 160 (80) |
| Serratia marcescens ATCC 13 880 | 10 | 20 |
| Klebsiella pneumoniae ATCC 10 031 | 10 (5) | 20 (10) |
| Proteus mirabilis | 10 | 10 |
| Proteus vulgaris | 5 | 10 |

Die Werte in Klammern bedeuten die Konzentrationen, bei denen Wachstumsminderung, aber noch kein völliger Waschstumsstillstand erkennbar ist.

Die akute Toxizität wurde durch perorale und subcutane Verabreichung beider Substanzen an weiße Laboratoriumsmäuse in steigenden Dosen bestimmt. Die $LD_{50}$ ist die Dosis, nach deren Verabreichung 50% der Tiere innerhalb von 8 Tragen verstarben. Die beiden Substanzen zeigten dabei eine $LD_{50}$ von über 5 g/kg; bei 5 g/kg verstarben bei beiden Substanzen keine Tiere. Nach subcutaner Injektion besitzen beide Substanzen eine $LD_{50}$ von über 1000 mg/kg; sie sind damit für die Praxis völlig untoxisch.

Beispiele zur Herstellung der Ausgangsprodukte:

### Beispiel A

*Spectinomycin-benzyloxim*

6 g (0,012 Mol) Spectinomycin-dihydrochlorid-pentahydrat und 3 g O-Benzylhydroxylamin werden in 25 ml Wasser und 25 ml Methanol gelöst. Die Lösung wird über Nacht gerührt, eingedampft, der Rückstand in wenig absolutem Äthanol gelöst, und mit Äther bis zur Trübung versetzt. Man erhält 5,0 g (83% der Theorie) farbloses Pulver vom Zersetzungspunkt 175°C.

Rf: 0,8 (Kieselgel, Chloroform/Methanol/konz. Ammoniak = 20:20:3)
NMR-Spektrum (Lösungsmittel $CD_3OD$)

ppm: 1,3 (Dublett, 3H ≙ 2—$CH_3$—)

2,8 (Dublett, 6H ≙ —N—$CH_3$)

4,65 (Singlett, 1H ≙ 10aH)

5,2 (Singlett, 2H ≙ Benzyl-$CH_2$)

7,4 (Singlett, 5H ≙ Phenyl)

Die freie Verbindung erhält man, indem man die wässrig-methanolische Lösung des Dihydrochlorids mit einem Ionenaustauscher (Dowex 2 x 8 ($OH^-$-Form) bis zum bleibenden pH-Wert von 10.4 versetzt.

Farblose Kristalle vom Schmelzbereich 86—106°C.

Auf dieselbe Weise wurden erhalten

a) 6,8-Bisbenzyloxycarbonyl-spectinomycinoxim aus 6,8-Bisbenzyloxycarbonylspectinomycin und Hydroxylamin

Rf: 0,38 (Kieselgel, Chloroform/Methanol = 9:1)
$C_{30}H_{37}N_3O_{11}$ (615,6)

| | | | |
|---|---|---|---|
| Ber.: | C 58,52 | H 6,06 | N 6,82 |
| Gef.: | 58,00 | 6,21 | 6,65 |

Das Ausgangsmaterial 6,8-Bisbenzyloxycarbonylspectinomycin ist literaturbekannt (J. Amer. chem. Soc. *85*, 2657 (1963)).

b) 6,8-Bis-benzyloxycarbonylspectinomycinmethyloxim aus 6,8-Bis-benzyloxycarbonylspectinomycin und O-Methyl-hydroxylamin,

Rf: 0,40 (Kieselgel; Chloroform/Methanol = 9:1).

c) Spectinomycin-benzhydrazon-dihydrochlorid aus Spectinomycin-dihydrochlorid-pentahydrat und Benzoylhydrazin,

Zersetzungspunkt: 180°C,
Rf: 0,35 (Cellulose, Butanol/Methanol/Wasser = 90:25:20).

d) Spectinomycin-acethydrazon-dihydrochlorid aus Spectinomycin-dihydrochlorid-pentahydrat und Acetylhydrazin,

Zersetzungsbereich: 160—180°C,
Rf: 0,29 (Cellulose, Butanol/Methanol/Wasser = 90/40/20).

Beispiel B

*6,8-Bis-β,β,β-trichloräthoxycarbonylspectinomycinbenzyloxim*

6 g (0,008 Mol) 6,8-Bis-β,β,β-trichloräthoxycarbonylspectinomycin und 1,5 g (0,01 Mol) O-Benzylhydroxylamin-hydrochlorid werden in 40 ml Dioxan und 40 ml Wasser gelöst. Durch Zugabe von 4n-Natronlauge wird ein pH-Wert von 3 bis 4 eingestellt. Nach 18-stündigem Rühren bei Raumtemperatur wird in 150 ml Wasser eingerührt und mit Essigester extrahiert. Die organische Phase wird getrocknet und eingedampft. Man erhält 6 g farbloses Produkt (93% der Theorie) Rf: 0,30 (Kieselgel, Chloroform/Methanol = 9:1)

NMR (Lösungsmittel Deuterochloroform)
ppm: 1,35 Dublett 2H (2—$CH_3$)

3,15 Dublett 6H (—N—$CH_3$)

4,7 Singlett 1H (10aH)

4,8 verbr. Singlett 2H (—$CH_2$—$CCl_3$)

5,2 Singlett 2H (Benzyl-$CH_2$)

7,4 Singlett 5H (Phenyl)

Das Ausgangsmaterial 6,8-Bis-β,β,β-trichloräthoxycarbonylspectinomycin läßt sich nach der in J. Antibiotics XXVIII, S. 140 (1975) für das 6,8-Bisbenzyloxycarbonyl-4-dihydro-spectinomycin angegebenen Methode aus Spectinomycin und Chlorameisensäure-β,β,β-trichloräthylester darstellen.

Rf: 0,26 (Kieselgel, Chloroform/Methanol = 11:1)

Auf dieselbe Weise wurden die folgenden Verbindungen erhalten:

a) 6,8-Bis-p-methoxybenzolsulfonylspectinomycin-oxim aus 6,8-Bis-p-methoxybenzolsulfonylspectinomycin und Hydroxylamin,

Rf: 0,32 (Kieselgel, Chloroform/Methanol = 11:1).

Das Ausgangsmaterial 6,8-Bis-p-methoxybenzolsulfonylspectinomycin wurde, wie oben angegeben, aus p-Methoxybenzolsulfochlorid und Spectinomycin erhalten.

Rf: 0,40 (Kieselgel, Chloroform/Methanol = 9:1)

b) 6,8-Bis-isobornyloxycarbonylspectinomycin-benzyloxim aus 6,8-Bis-isobornyloxycarbonylspectinomycin und O-Benzylhydroxylamin,

Rf: 0,55 (Kieselgel, Chloroform/Methanol = 10:1),
Schmelzpunkt: 120°C (Zersetzung).

Das Ausgangsmaterial wurde, wie oben angegeben, aus Spectinomycin und Isobornyloxycarbonylchlorid hergestellt;

Rf: 0,42 (Kieselgel, Chloroform/Methanol = 10:1)

Beispiel C

*6,8-Bis-benzyloxycarbonylspectinomycylamin*

1,85 g (0,003 Mol) 6,8-Bis-benzyloxycarbonylspectinomycyin und 2,4 g Ammoniumnitrat werden in 15 ml absolutem Methanol gelöst und 15 Minuten bei 40°C gerührt. Anschließend werden innerhalb von 3 Minuten bei 20°C 0,131 g Natriumcyanborhydrid portionsweise zugegeben. Nach 30 Minuten bei Raumtemperatur wird abgesaugt, das Filtrat in 70 ml einer gesättigten Kochsalz-Lösung eingerührt, mit Essigester extrahiert, der Extrakt getrocknet und eingedampft. Der Rückstand wird mit Äther verrieben und abgesaugt. Dieser Rückstand (1,2 g) wird an Kieselgel chromatographiert (Chloroform/Methanol = 5:1). Man erhält 0,6 g eines farblosen Pulvers vom Zersetzungsbereich 110—130°C.

$C_{30}H_{39}N_3O_{10}$ (601,66)

| | | | |
|---|---|---|---|
| Ber.: | C 58,89 | H 6,53 | N 6,57 |
| Gef.: | 59,10 | 6,99 | 6,76 |

Massenspektrum der silylierten Substanz:

$M^+$: 817 = 601 + 3×72 (3 Silylreste)
745 = 601 + 2×72 (2 Silylreste)
Rf: 0,47 (Kieselgel, Chloroform/Methanol = 5:1)

Auf dieselbe Weise wurden dargestellt:

a) 6,8-Bis-p-methoxybenzolsulfonylspectinomycylamin aus 6,8-Bis-p-methoxybenzolsulfonylspectinomycin, Ammoniumnitrat und Natriumcyanborhydrid,
Rf: 0,55 (Kieselgel, Chloroform/Methanol = 9:1)

b) 6,8-Bis-β,β,β-trichloräthoxycarbonylspectinomycylamin aus 6,8-Bis-β,β,β-trichloräthoxycarbonylspectinomycin, Ammoniumnitrat und Natriumcyanborhydrid, Rf: 0,41 (Kieselgel, Chloroform/Methanol = 9:2) Massenspektrum der silylierten Verbindung: $M^+$: 969 = 681 + 4×72 (4 Silylreste).
Berechnetes Molekulargewicht: 684,18.

c) 6,8-Bis-isobornyloxycarbonylspectinomycylamin aus 6,8-Bis-isobornyloxycarbonylspectinomycin, Ammoniumnitrat und Natriumcyanborhydrid.
Massenspektrum der silylierten Substanz:

$M^+$: 909 = 693 + 3 × 72 (3 Silylreste)

837 = 693 + 2×72 (2 Silylreste)

693 = berechnetes Molekulargewicht

Schmelzpunkt: 160°C (Zersetzung)

d) 6,8-Bis-4-methoxybenzyloxycarbonylspectinomycylamin aus 6,8-Bis-4-methoxybenzyloxycarbonylspectinomycin, Ammoniumnitrat und Natriumcyanborhydrid.

Rf: 0,29 (Kieselgel, Chloroform/Methanol 5:1)

Massenspektrum der silylierten Verbindung:

$M^+$: 877 = 661 + 3×72 (3 Silylreste)

Berechnetes Molekulargewicht: 661,6.

Beispiel D

*6,8-Bis-isobornyloxycarbonyl-spectinomycylamin*

7,98 g (0,01 Mol) 6,8-Bis-isobornyloxycarbonyl-spectinomycinbenzyloxim, gelöst in 150 ml 3%iger äthanolischer Salzsäure, werden in Gegenwart von 8 g Platinoxid mit Wasserstoff bei 25°C reduziert.

(Wasserstoffdruck: 5 Atmosphären; Reaktionszeit: 62 Stunden).

Nach beendeter Reduktion wird vom Katalysator abgesaugt und das Äthanol im Vakuum abdestilliert.

Man löst den verbleibenden festen Rückstand in Wasser, stellt den pH-Wert der Lösung auf 3,5 ein und trennt durch Extraktion mit Äther die Nebenprodukte ab. Anschließend wird der pH-Wert der Lösung auf 7,0 erhöht und erneut mit Äther extrahiert. Nach Trocknen mit Natriumsulfat und Abziehen des Äthers wird das gewünschte Produkt in Form eines weißen Pulvers erhalten.

Ausbeute: 4,1 g (59% der Theorie),
F.: 160°C (Zersetzung)

$C_{36}H_{59}N_3O_{10}$ (693,97)

| | | | |
|---|---|---|---|
| Ber.: | C 49,41 | H 6,12 | N 8,23 |
| Gef.: | 49,29 | 6,25 | 8,37 |

Das Hydrochlorid läßt sich erhalten, in dem man die äthanolische Lösung der freien Base mit ätherischer Salzsäure behandelt.

Schmelzpunkt: 182—185°C.

Auf dieselbe Weise wurde erhalten

a) 6,8-Bis-β,β,β-trichloräthoxycarbonylspectinomycylamin aus 6,8-Bis-β,β,β-trichloräthoxycarbonylspectinomycin-benzyloxim und Platindioxid.

Rf: 0,41 (Kieselgel, Chloroform/Methanol = 9:2)

Massenspektrum der silylierten Verbindung:

$M^+$: 969 = 681 + 4×72 (4 Silylreste)

897 = 681 + 3×72 (3 Silylreste)

825 = 681 + 2×72 (2 Silylreste)

Berechnetes Molekulargewicht: 684,18

b) 6,8-Bis-p-methoxybenzolsulfonylspectinomycylamin aus 6,8-Bis-p-methoxybenzolsulfonylspectinomycinoxim und Platindioxid.

Rf: 0,55 (Kieselgel, Chloroform/Methanol = 9:1)

Beispiele zur Herstellung des Endproduktes:

Beispiel 1

*4-R-Spectinomycylamin-trihydrochlorid*

500 mg 6,8-Bisbenzyloxycarbonylspectinomycylamin (R-Form) werden in 25 ml 3,4%iger

äthanolischer Salzsäure mit 500 mg 20%iger Palladiumkohle zwei Stunden bei Raumtemperatur in einer Schüttelente hydriert. Der Katalysator wird abfiltriert, das Filtrat auf 5 ml eingeengt und der Rückstand mit 100 ml Äther versetzt. Man erhält 230 mg (65% der Theorie) farbloses Pulver vom Schmelzpunkt 189—194°C.

Rf: 0,5 (Kieselgel, Chloroform/Methanol/konz. Ammoniak = 40/40/15)

Massenspektrum der silylierten Verbindung:

$M^+$ 693 = 333 + 5×72 (5 Silylreste)

621 = 333 + 4×72 (4 Silylreste)

549 = 333 + 3×72 (3 Silylreste)

477 = 333 + 2×72 (2 Silylreste)

Berechnetes Molekulargewicht der freien Base 333.

## Beispiel 2

*4-R-Spectinomycylamin-trihydrochlorid*

5 g Spectinomycinbenzyloxim-dihydrochlorid werden in 500 mg 3,4%iger äthanolischer Salzsäure in Gegenwart von 6,25 g Platindioxid bei Raumtemperatur und 15 at 30 Stunden hydriert. Der Katalysator wird abfiltriert, das Filtrat auf 100 ml eingeengt. Die erhaltenen Kristalle werden abfiltriert.

Ausbeute: 2,5 g (59% der Theorie)

Massenspektrum der silylierten Verbindung:

$M^+$ 621 = 333 + 4×72 (4 Silylreste)

Berechnetes Molekulargewicht der freien Base 333.

Die freie Base läßt sich erhalten, indem man die wässrige Lösung des Hydrochlorids mit Ionenaustauscher (Dowex 2 × 8 $OH^-$Form) auf einen pH-Wert von 10.9 einstellt und die wässrige Lösung im Hochvakuum eindampft.

F.: 114—117°C (Zersetzung).

## Beispiel 3

*4-S-Spectinomycylamin-trihydrobromid*

1 g (0,00145 Mol) 6,8-Bis-isobornyloxycarbonyl-spectinomycylamin (S-Form) werden mit 10 ml einer 20%igen Lösung von Bromwasserstoff in Eisessig bei 20°C versetzt.

Nach 2 Minuten gibt man zu der Reaktionsmischung 100 ml absoluten Äther. Die ausgefallenen Kristalle werden abzentrifugiert, 3 mal mit absolutem Äther gewaschen und getrocknet.

Die gewünschte Verbindung liegt sofort in reiner Form vor.

Ausbeute: 0,65 g (78% der Theorie)

F.: 208—212°C (Zersetzung)

$C_{14}H_{27}N_3O_6 \cdot 3HBr$ (576,14)

| | | | | |
|---|---|---|---|---|
| Ber.: | C 29,18 | H 5,24 | N 7,29 | Br 41,60 |
| Gef.: | 29,64 | 5,39 | 7,01 | 41,35 |

## Beispiel 4

*4-R-Spectinomycyl-amin-trihydrochlorid*

Eine Lösung von 6,84 g (0,01 Mol) 6,8-Bis-trichloräthoxycarbonylspectinomycylamin (R-Form) in 200 ml 70%igem Methanol wird nach Zusatz von 14 g Zinkpulver zum Rückfluß erhitzt.

Die Reaktionsmischung wird abgesaugt und das Filtrat im Vakuum eingedampft. Man löst den Rückstand in wenig Methanol und reinigt durch Säulenchromatographie.

(Adsorbens: Kieselgel G;
Eluens: Chloroform/Methanol/konz.wäßriges Ammoniak = 20:20:5)

Die das gewünschte Produkt enthaltenden Fraktionen werden vereinigt und schonend im Vakuum eingedampft.

Der verbleibende Rückstand wird in 0,1n-Salzsäure gelöst und gefriergetrocknet. Die gewünschte Substanz wird als weißes Pulver erhalten.

Ausbeute: 2,1 g (47% der Theorie)
F.: 189—194°C (Zers.)

$C_{14}H_{27}N_3O_6 \cdot 3\ HCl$ (442,79)

| | | | | |
|---|---|---|---|---|
| Ber.: | C 37,98 | H 6,83 | N 9,49 | Cl 24,02 |
| Gef.: | 38,12 | 6,99 | 9,27 | 23,62 |

Beispiel 5

*4-R-Spectinomycylamin-trihydrochlorid*

500 mg 6,8-Bis-p-methoxybenzolsulfonyl-spectinomycylamin (R-Form) werden in 3 ml Methansulfonsäure und 0,3 ml Anisol gelöst und 3 Stunden bei 20°C stehen gelassen. Das Gemisch wird in 20 ml Äther eingerührt, das ausgeschiedene ölige Produkt mehrmals mit Äther digeriert, in wenig Wasser gelöst und durch Säulenchromatographie gereinigt.

($SiO_2$, Chloroform/Methanol/konz. Ammoniak = 20:20:5)

Das erhaltene Produkt wird in der berechneten Menge n/100-Salzsäure gelöst, und bei 0°C im Hochvakuum eingedampft. Das Produkt wird mit Äther gewaschen und getrocknet.

Ausbeute: 50 mg (25% der Theorie)

Schmelzpunkt: 187—190°C.

Beispiel 6

*4R-Spectinomycylamin-trihydrochlorid*

2 g 6,8-Bis-4-methoxybenzyloxycarbonyl-spectinomycylamin werden in 10 ml Dioxan gelöst und bei 0°C mit 10 ml 4n-Chlorwasserstoff in Dioxan versetzt. Es wird 45 Minuten gerührt, abfiltriert und mit Äther gewaschen. 1,2 g farbloses Pulver.

F.: 186—188°C (Zersetzung)

Massenspektrum der silylierten Verbindung.

$M^+ = 765/693/621 = 333 + (4—6) \times 72.$

Die neuen Verbindungen der allgemeinen Formel I lassen sich zur pharmazeutischen Anwendung in die üblichen pharmazeutischen Zubereitungsformen einarbeiten. Die Einzeldosis beträgt bei Erwachsenen 100 bis 2000 mg, vorzugsweise 500 bis 1000 mg, die Tagesdosis 200 bis 6000 mg, vorzugsweise 1000 bis 2000 mg.

Die nachfolgenden Beispiele beschreiben die Herstellung einiger pharmazeutischer Zubereitungsformen:

Beispiel I

*Trockenampullen mit 1 g 4R-Spectinomycylamin-trihydrochlorid*

Zusammensetzung:

1 Trockenampulle enthält
Wirksubstanz 1000,0 mg, die Lösungsmittelampulle 5 ml Aqua pro Inj. und 200 mg Kochsalz.

Herstellungsverfahren:

In die Trockenampulle wird eine lyophilisierbare Lösung des Wirkstoffes in Wasser eingefüllt, gefriergetrocknet und keimfrei verschlossen. Das Lösungsmittel wird mit Kochsalz isotonisch eingestellt und in die Ampulle keimfrei abgefüllt und sterilisiert. Zur Anwendung wird der Inhalt der Trockenampulle mit dem Lösungsmittel gelöst und injiziert.

Beispiel II

*Ampulle mit 1 g 4R-Spectinomycylamin-trihydrochlorid*

Zusammensetzung:

1 Ampulle enthält
1 g Wirkstoff,
200 mg Kochsalz,
Aqua pro inj. ad 5 ml.

Kochsalz und Wirksubstanz wird im Wasser gelöst, die Lösung wird sterilfiltriert und unter aseptischen Bedingungen in Ampullen abgefüllt.

Die pharmazeutischen Zubereitungsformen mit den weiter oben angegebenen Dosierungen werden zur Prophylaxe und Chemotherapie von lokalen und systemischen Infektionen in der Human- und Tiermedizin verwendet. Als Krankheiten, die durch die erfindungsgemäßen Verbindungen bzw. durch die, diese enthaltende Zubereitungsformen verhindert bzw. geheilt werden können, seien beispielsweise solche der Atmungswege, des Rachenraumes und der Harnwege genannt.

**Patentansprüche**

1. 4-Spectinomycylamin der Formel

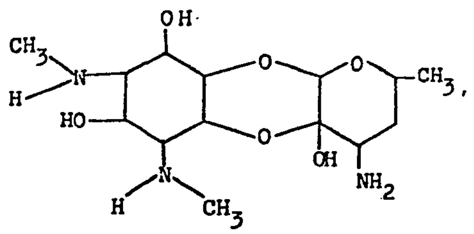

(I)

und dessen physiologisch verträgliche Säureadditionssalze mit anorganischen oder organischen Säuren.

2. Verfahren zur Herstellung von 4-Spectinomycylamin der Formel

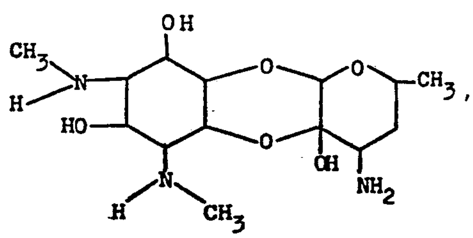

(I)

und von dessen Säureadditionssalzen mit anorganischen oder organischen Säuren, dadurch gekennzeichnet, daß

a) ein Spectinomycinderivat der allgemeinen Formel

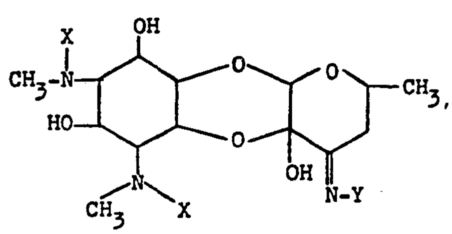

(II)

in der X ein Wasserstoffatom oder eine aus der Peptidchemie bekannte, durch Reduktion leicht abspaltbare organische Gruppe bedeutet und

Y eine Hydroxygruppe, eine Alkoxygruppe mit 1 bis 10 Kohlenstoffatomen, die Phenalkoxygruppe mit insgesamt 7 bis 12 Kohlenstoffatomen oder eine Gruppe der Formel

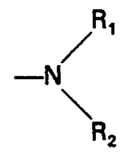
,

in der $R_1$ und $R_2$ Wasserstoffatome, Alkylgruppen mit 1 bis 6 Kohlenstoffatomen, Phenalkylgruppen mit insgesamt 7 bis 10 Kohlenstoffatomen oder die Phenylgruppe darstellen, wobei $R_1$ auch eine aliphatische Acylgruppe mit 1 bis 10 Kohlenstoffatomen oder die Benzoylgruppe sein kann, sofern dann $R_2$ die anderen vorstehend genannten Bedeutungen innehat, oder eine Gruppe der Formel

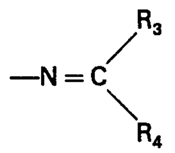
,

in der $R_3$ und $R_4$ Wasserstoffatome, Alkylgruppen mit 1 bis 6 Kohlenstoffatomen, Phenalkylgruppen mit 7 bis 10 Kohlenstoffatomen oder die Phenylgruppe darstellen, wobei $R_3$ und $R_4$ zusammen mit dem dazwischenliegenden Kohlenstoffatom auch einen 5- bis 8-gliedrigen carbocyclischen Ring bilden kann, bedeutet, in Gegenwart von Metallkatalysatoren in einem Lösungsmittel vorteilhafterweise in Gegenwart von anorganischen oder organischen Säuren bei Temperaturen zwischen 0° und 100°C und Drucken zwischen 1 und 100 at hydriert wird oder

b) aus einem 4-Spectinomycylaminderivat der allgemeinen Formel

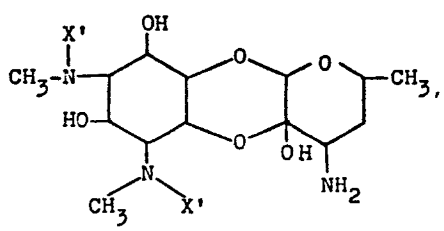
(III)

in der X' eine aus der Peptidchemie bekannte, durch Behandeln mit Säuren, Basen oder durch Reduktion leicht abspaltbare organische Gruppe bedeutet, die Gruppe X' mittels katalytischer Hydrogenolyse in wässerig-organischen oder organischen Lösungsmitteln, mittels nascierendem Wasserstoff, mittels reduktiver Spaltung mit Natrium in flüssigem Ammoniak oder durch acidolytische Spaltung mittels Säuren oder durch die Einwirkung von Basen, abgespalten wird und, gewünschtenfalls, anschließend die so erhaltene Verbindung der Formel I mittels anorganischen oder organischen Säuren in ihre Säureadditionssalze übergeführt wird.

3. Verfahren gemäß Anspruch 2a, dadurch gekennzeichnet, daß als Metallkatalysatoren feinverteiltes Platin, Palladium oder Platindioxid, als Lösungsmittel Wasser, Alkohole, Carbonsäuren, Dioxan, Tetrahydrofuran, oder Gemische dieser Lösungsmittel verwendet werden und die Reduktion in Gegenwart anorganischer oder organischer Säuren durchgeführt wird.

4. Verfahren gemäß Anspruch 2b, dadurch gekennzeichnet, daß die katalytische Hydrogenolyse in wässerig-organischen oder rein organischen Lösungsmitteln, z.B. in Alkoholen, Essigsäure, Dimethylformamid, mittels Edelmetallkatalysatoren, wie Palladiumschwarz, Palladiumkohle oder Palladium auf Bariumsulfat oder Platin auf Kohle, die acidolytische Spaltung mittels Halogenwasserstoffsäuren, Trifluoressigsäure oder organischer Sulfonsäuren, in Wasser, Eisessig, Chloroform, Alkoholen oder in Gemischen hiervon, die basische Spaltung mittels Alkalihydroxyde oder Alkalialkoholate und die Spaltung mittels nascierendem Wasserstoff durch die Einwirkung von Zink auf Methanol oder Essigsäure erreicht wird.

5. Arzneimittel bestehend aus dem 4-Spectinomycylamin der Formel I gemäß Anspruch 1 oder dessen physiologisch verträglichen Säureadditionssalzen mit anorganischen oder organischen Säuren und übrigen Träger- und Hilfsmitteln.

6. Die Verwendung des 4-Spectinomycylamins der Formel I gemäß Anspruch 1 oder von dessen physiologisch verträglichen Säureadditionssalzen mit anorganischen oder organischen Säuren zur Herstellung auf nichtchemischem Weg eines Arzneimittels mit antibakterieller Wirkung.

**Revendications**

1. 4-spectinomycylamine de formule

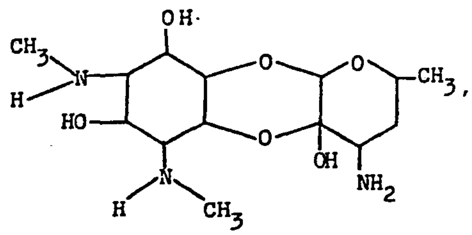

(I)

et ses sels d'addition physiologiquement acceptables avec des acides minéraux ou organiques.

2. Procédé pour la préparation de la 4-spectinomycylamine de formule

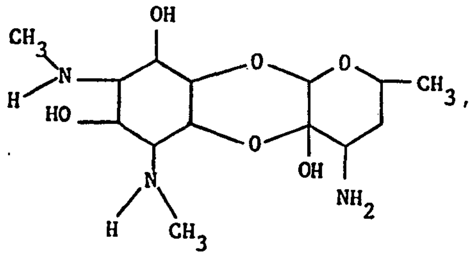

(I)

et de ses sels d'addition avec des acides minéraux ou organiques, caractérisé en ce que

a) on hydrogène un dérivé de spectinomycine de formule générale

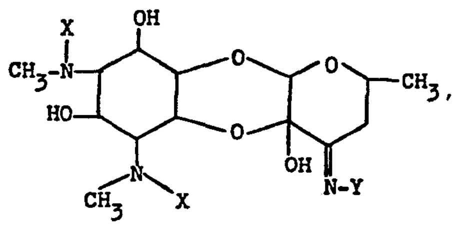

(II)

dans laquelle X représente un atome d'hydrogène ou un groupe organique facilement clivable par réduction, connu dans la chimie des peptides et

Y représente un groupe hydroxy, un groupe alcoxy avec 1 à 10 atomes de carbone, le groupe phénylalcoxy avec en tout 7 à 12 atomes de carbone ou un groupe de formule

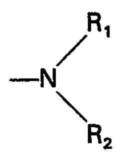

,

dans laquelle $R_1$ et $R_2$ représentent des atomes d'hydrogène, des groupes alcoyle avec 1 à 6 atomes de carbone, des groupes phénylalcoyle avec en tout 7 à 10 atomes de carbone ou le groupe phényle, $R_1$ pouvant également être un groupe acyle aliphatique avec 1 à 10 atomes de carbone ou le groupe benzoyle, dans la mesure où alors $R_2$ possède les autres significations mentionnées plus haut, ou un groupe de formule

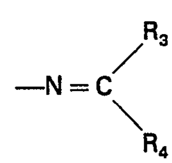

,

dans laquelle $R_3$ et $R_4$ représentent des atomes d'hydrogène, des groupes alcoyle avec 1 à 6 atomes de carbone, des groupes phénylalcoyle avec 7 à 10 atomes de carbone ou le groupe phényle, $R_3$ et $R_4$ pouvant également former ensemble avec l'atome de carbone intermédiaire un cycle carboné comportant 5 à 8 membres, en présence de catalyseurs métalliques dans un solvant avantageusement en présence d'acides minéraux ou organiques à des températures comprises entre 0° et 100°C et sous des pressions comprises entre 1 et 100 atm. ou

b) à partir d'un dérivé de 4-spectinomycylamine de formule générale

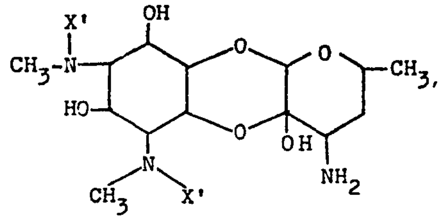

(III)

dans laquelle X' représente un groupe organique facilement clivable par traitement avec des acides, des bases ou par réduction, connu dans la chimie des peptides, on clive le groupe X' au moyen d'une hydrogénolyse catalytique dans des solvants hydro-organiques ou organiques, à l'aide d'hydrogène naissant, à l'aide d'un clivage réducteur par le sodium dans l'ammoniac liquide ou par clivage acidolytique au moyen d'acides ou par action de bases, et, si on le désire, on transforme ensuite le composé ainsi obtenu de formule I en ses sels d'addition avec des acides, au moyen d'acides minéraux ou organiques.

3. Procédé suivant la revendication 2*a*, caractérisé en ce que l'on utilise en tant que catalyseurs métalliques du platine, du palladium ou du dioxyde de platine finement divisé comme solvant de l'eau, des alcools, des acides carboxyliques, du dioxanne, du tétrahydrofuranne, ou des mélanges de ces solvants, et en ce que la réduction est effectuée en présence d'acides minéraux ou organiques.

4. Procédé suivant la revendication 2*b*, caractérisé en ce que l'hydrogénolyse catalytique est obtenue dans des solvants hydro-organiques ou purement organiques, par exemple dans des alcools, l'acide acétique, le diméthylformamide, au moyen de catalyseurs à base de métaux précieux, comme le noir de palladium, le palladium sur charbon ou le palladium sur sulfate de baryum ou le platine sur charbon, le clivage acidolytique au moyen d'acides halohydriques, d'acide trifluoroacétique ou d'acides sulfoniques organiques, dans l'eau, l'acide acétique cristallisable, le chloroforme, les alcools ou des mélanges de ces solvants, le clivage basique au moyen d'hydroxydes alcalins ou d'alcoolates alcalins et le clivage au moyen d'hydrogène naissant par action du zinc sur le méthanol ou l'acide acétique.

5. Produit pharmaceutique constitué de la 4-spectinomycylamine de formule I selon la revendication 1, ou de ses sels d'addition physiologiquement acceptables avec des acides minéraux ou organiques et pour le reste d'excipients et d'adjuvants.

6. L'utilisation de la 4-spectinomycylamine de formule I selon la revendication 1, ou de ses sels d'addition physiologiquement acceptables avec des acides minéraux ou organiques pour la préparation par une voie non chimique d'un produit pharmaceutique à activité antibactérienne.

**Claims**

1. 4-Spectinomycylamine of formula

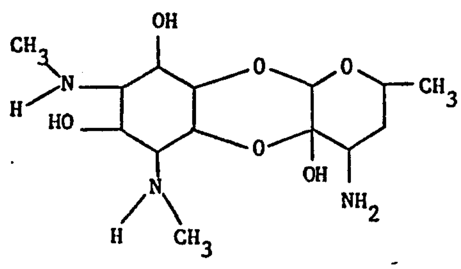

(I)

and its physiologically compatible acid addition salts with inorganic or organic acids.

2. Process for the preparation of 4-spectinomycylamine of formula

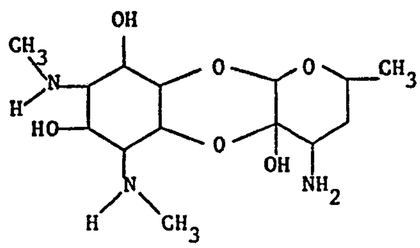

(I)

and of its acid addition salts with inorganic or organic acids, characterised in that

a) a spectinomycine derivative of general formula

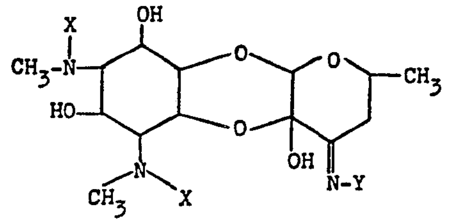

(II)

in which X represents a hydrogen atom or an organic group known from peptide chemistry and easily separable by reduction and Y represents a hydroxy group, an alkoxy group with 1 to 10 carbon atoms, the phenalkoxy group with altogether 7 to 12 carbon atoms or a group of formula

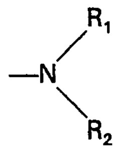
,

in which $R_1$ and $R_2$ represent hydrogen atoms, alkyl groups with 1 to 6 carbon atoms, phenalkyl groups with altogether 7 to 10 carbon atoms or the phenyl group, whereby $R_1$ can also be an aliphatic acyl group with 1 to 10 carbon atoms or the benzoyl group, if $R_2$ then has the other above-mentioned meanings, or a group of formula

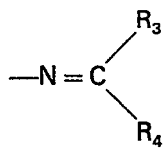
,

in which $R_3$ and $R_4$ represent hydrogen atoms, alkyl groups with 1 to 6 carbon atoms, phenalkyl groups with 7 to 10 carbon atoms or the phenyl group, whereby $R_3$ and $R_4$ together with the carbon atom lying therebetween can also form a 5- to 8-membered carbocyclic ring, is hydrogenated in the presence of metal catalysts in a solvent advantageously in the presence of inorganic or organic acids at temperatures of between 0° and 100°C and pressures of between 1 and 100 atm or

b) from a 4-spectinomycylamine derivative of general formula

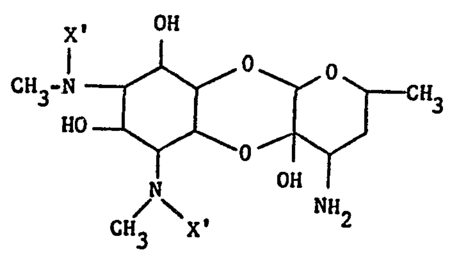

(III)

in which X′ represents an organic group known from peptide chemistry and easily separable by treatment with acids, bases or by reduction, the group X′ is separated by means of catalytic hydrogenolysis in aqueous-organic or organic solvents, by means of nascent hydrogen, by means of reductive separation with sodium in liquid ammonia or by acidolytic separation by means of acids or by the action of bases and, if desired, the compound of formula I thus obtained is subsequently converted by means of inorganic or organic acids into its acid addition salts.

3. Process according to claim 2a, characterised in that the metal catalysts used are finely dispersed platinum, palladium or platinum dioxide and the solvents used are water, alcohols, carboxylic acids, dioxan, tetrahydrofuran or mixtures of these solvents and in that the reduction is effected in the presence of inorganic or organic acids.

4. Process according to claim 2b, characterised in that the catalytic hydrogenolysis is effected in aqueous-organic or purely organic solvents, for examples in alcohols, acetic acid, dimethylformamide, by means of precious metal catalysts such as palladium black, palladium charcoal or palladium on barium sulphate or platinum on charcoal, the acidolytic separation is effected by means of hydrohalic acids, trifluoroacetic acid or organic sulphonic acids in water, glacial acetic acid, chloroform, alcohols or mixtures thereof, the basic separation is effected by means of alkali hydroxides or alkali alcoholates and the separation by means of nascent hydrogen is effected by the action of zinc on methanol or acetic acid.

5. Pharmaceuticals consisting of the 4-spectinomycylamine of formula I according to claim 1 or its physiologically compatible acid addition salts with inorganic or organic acids and other carriers and excipients.

6. The use of the 4-spectinomycylamine of formula I according to claim 1 or of its physiologically compatible acid addition salts with inorganic or organic acids for the preparation in a non-chemical way of a pharmaceutical with anti-bacterial activity.